# EUROPEAN PATENT SPECIFICATION

(11) **EP 1 317 917 B1**
(45) Date of publication and mention of the grant of the patent: **09.01.2008**
(21) Application number: 02257733.2
(22) Date of filing: 07.11.2002
(51) Int. Cl.: A61K 8/26, A61K 8/35, A61K 8/41, A61K 8/46, A61K 8/73, A61Q 5/02, A61Q 5/10

(54) **Hair colouring composition comprising clay**
Haarfärbezusammensetzung enthaltend Ton
Compositions de coloration capillaire comprenant de l'argile

(30) Priority: 04.12.2001 EP 01310130
(43) Date of publication of application: 11.06.2003
(73) Proprietor: UNILEVER PLC, London EC4P 4BQ (GB); UNILEVER N.V., 3013 AL Rotterdam (NL)
(72) Inventor: Baker, Mark Edward J. Unilever R & D Port Sunlight, Wirral, Merseyside, CH63 3JW (GB); Madden, Timothy John, Unilever R & D Port Sunlight, Wirral, Merseyside, CH63 3JW (GB)
(74) Representative: Newbould, Frazer Anthony

(56) References cited:
- EP-A- 0 823 250
- DE-A- 4 020 272
- FR-A- 2 549 721
- US-A- 4 402 698
- US-A- 5 902 591
- DATABASE WPI Section Ch, Week 197621 Derwent Publications Ltd., London, GB; Class A97, AN 1976-38920X XP002197917 & JP 51 041443 A (KOBAYAHI KOSE KK), 7 April 1976 (1976-04-07)
- PATENT ABSTRACTS OF JAPAN vol. 003, no. 087 (C-053), 25 July 1979 (1979-07-25) & JP 54 064644 A (SHISEIDO CO LTD), 24 May 1979 (1979-05-24)
- DATABASE WPI Section Ch, Week 197926 Derwent Publications Ltd., London, GB; Class D21, AN 1979-48186B XP002197918 & JP 54 063132 A (SHISEIDO CO LTD), 21 May 1979 (1979-05-21)

## Description

This invention relates to a method of producing a hair colouring composition, to a method of colouring hair using the composition and to the use of a clay.

Traditional hair colouring compositions (also called colorants) can be permanent, semi-permanent or temporary in nature.

Permanent colorants use oxidative dyes and generally comprise two components; a mixture of dye precursors and a developer solution that initiates the formation of coloured dye molecules and enables them to penetrate into the hair fibre. These systems take the form of a treatment which is applied every four to eight weeks to cover outgrowth. The treatment involves the mixing of the two components, the application of the mixture to the hair and a finite period of time during which the colorant is left on the hair to take effect. As such, permanent hair colouring requires a significant amount of time and effort from the consumer. Furthermore, the treatment is non-specific to hair and hence requires gloves to protect the hands from staining during use and permanent hair colourants can also lead to a significant amount of scalp staining if the consumer does not apply the product with great care.

Semi-permanent colorants are generally single component mixtures that contain dyes, especially nitro dyes, which because of their small size can penetrate the hair and impart colour. These products also require a finite amount of contact time with the hair and are , non-specific, again requiring the use of gloves and with the potential of adverse scalp staining.

Furthermore, permanent colorants and semi-permanent colorants cannot be used on a regular, particularly daily, basis due to the toxicity of the dyes and dye precursors.

Finally, temporary dyes are generally large molecules that interact with the hair surface via adsorption processes. However, these are also non-specific in colour delivery and in many cases are not water-fast. Temporary colours are usually leave on systems and as such require the user to apply the product and then wait some period of time until it has dried. This process is both time consuming and inconvenient since the product must be applied once the consumer has finished the normal hair care regimen. Temporary colourants also suffer the limitation of being easily rubbed off onto clothing and pillows.

It is desirable to produce hair colouring compositions that have reduced skin staining and a higher relative selectivity for colouring hair instead of skin. It would also be desirable to provide compositions that can be used on a daily basis, for example by delivery from a shampoo or conditioner. However, existing hair colouring technology cannot be directly applied to provide such compositions for the reasons given above, particularly skin staining.

It is known that clays can be used in hair colorant compositions.

For example, compositions for non-permanently or semi-permanently colouring hair are disclosed in US 5,110,318. The compositions contain from 5% to 25% of a clay and require the treated hair to be heated such that the composition dries to a hard and flaky state. The document exemplifies compositions in which an anionic or neutral dye is employed with an anionic clay. Furthermore, the document teaches away from using substantial amounts of cationic components in the compositions "as an excess amount will complex with the anionic colouring components" having the effect of "reducing the effectiveness of the clay-based compositions to colour hair" (column 6 of the document). There is no mention in the document of the problems of skin staining.

Temporary hair dye compositions containing clays are described in JP-A-11292744. Anionic or neutral dyes are used together with anionic clays.

Mechanistic studies have been carried out on combinations of anionic clays with cationic dyes. The term anionic clays and related terms, as used herein, refers to clays which are themselves anionic in nature ie, the clays themselves are negatively charged and are capable of exchanging cations. Examples of such studies are described in, for instance: Miyamoto et al, "Adsorption and aggregation of a cationic cyanine dye on layered clay minerals", Appl Clay Sci, 16, (3-4), 161-170, March 2000; Iwasaki et al, "Intercalation characteristics of 1,1'-diethyl-2,2'-cyanine and other cationic dyes in synthetic saponite: Orientation in the interlayer", Clay Miner, 48, (3), 392-399, June 2000; Arbeloa et al, "The hydrophobic effect on the adsorption of rhodamines in aqueous suspensions of smectites. The rhodamine 3B Laponite B system", Langmuir, 14, (16), 4566-4573, August 4 1998. There is no mention in these documents of the colouring of hair and none of the compositions disclosed in these documents contains components typically present in hair colouring compositions such as perfume and surfactant.

DE-A-4020272 discloses a pigment based on a layered double hydroxide. The pigment is said to have application in a number of areas, including cosmetics. However, there is no suggestion that the pigment could be used in compositions for treating hair and certainly no indication that the pigment could be used to dye hair in a rinse off composition. The compositions are clearly intended to provide colour to a particular product and it is not the intention to allow transfer of that colour to other materials, such as hair.

JP-A-54-064644 describes a red pigment for use in cosmetics which is obtained by adsorbing a reaction product of the cationic dye, Rhodamine B, with an anionic dye to a clay material. Again, there is no suggestion that the pigment could be used to colour hair in a rinse off composition. Instead, the aim of the compositions is stated as being to prevent colour transfer and to have reduced dyeability.

US 4402698 discloses a two stage process for dyeing hair. In the first stage, a barrier material is applied to protect the skin and a dye is applied in a subsequent second stage. The barrier material, which may be a clay, acts as a physical barrier to prevent the dye from contacting the scalp. There is no teaching of a single composition that contains both a dye and a clay.

There remains a need for colouring compositions that act in a more selective manner than conventional compositions ie, they cause relatively less colouring of the skin compared to the hair. It is preferable that the compositions have the further advantages of being able to impart colour to hair from a hair conditioner or shampoo on a daily basis, with good water-fastness, preferably with a degree of reversibility (ie, the dye can be washed off with shampoo).

Accordingly, the present invention provides a method of colouring hair comprising applying to the hair a rinse off hair colouring composition comprising:
(i) a clay having a net positive or negative charge at its surface;
(ii) an agent capable of imparting a colour to hair; and
(iii) a perfume and/or a surfactant,
wherein the agent has a net charge which is opposite from the charge on the surface of the clay.

In another aspect, the invention provides a method of producing a hair colouring composition of the invention which comprises dispersing the clay in an aqueous liquid to form a dispersion and then bringing the agent into contact with the dispersion.

Further provided by the invention in another aspect is the use of a clay to reduce the amount that an agent capable of imparting a colour to hair colours skin relative to the amount that the agent colours hair, wherein the clay has a net charge at its surface and the agent has a net charge which is opposite from that of the clay.

The invention goes against the conventional teaching, for example as disclosed in US 5,110,318, that anionic and cationic components should not be used together. Indeed, the Laporte Technical Directory for the clay Laponite, "Laponite: The Performance Enhancer" states on page 7 that "Laponite products are anionic, and their use in formulations containing cationic compounds is not recommended".

Surprisingly, in the present invention, it is possible to deliver colour to hair whilst reducing skin staining. It is unexpected that skin staining can be reduced whilst still achieving good (and, in some embodiments of the invention, better) delivery of colour to the hair, particularly when the teaching in the art is that clays should not be used with an agent having an opposite charge from that of the clay where delivery of that agent (eg, to hair) is required.

The invention may have one or more of the further advantages of:
(i) allowing colour to be delivered on a daily basis, for example, from a hair conditioner or shampoo;
(ii) achieving good water-fastness of the colour on the hair ie, good resistance to rinsing with water;
(iii)achieving more effective reversible colouring of the hair compared to the dye used alone (ie, the colour can, if desired, be washed off to some extent with shampoo);
(iv) reducing any toxicity associated with free dye molecules;
(v) imparting good lubrication properties to the hair;
(vi) reducing the need for gloves when the composition is applied; and
(vii) getting reduced rub off of the dye onto fabrics that come into contact with the hair, such as clothes and pillows.

The Compositions to be used in a method of the invention may be intended to be primarily hair colourant products, optionally having secondary benefits such as hair conditioning and/or cleaning properties. Alternatively, the compositions may be intended to be primarily for use in cleaning and/or conditioning hair with colouring representing a secondary benefit. Compositions of the invention may be used to colour all or only part of the hair. For example, the compositions may be used to impart streaks or highlights to hair.

The clay which is used in the compositions of the invention can be any clay material known in the art. Suitable clays include natural clays, synthetic clays and chemically modified clays. In general, the term clay refers to a composition comprising fine particles which have a net electrostatic (ie, positive or negative charge) on at least one surface. Preferably, the clay comprises a hydrous silicate of aluminium, magnesium or iron.

Preferably, the clay has a layered structure. In the compositions of the invention, the clay is advantageously present in the form of a dispersion (for example a sol or gel) or suspension of the clay particles.

Clays of the invention may be anionic or cationic clays, ie, they may have a net charge on the surface of the clay that is negative or positive, respectively, depending on the nature of the dye which is used. As mentioned above, the term anionic clays and related terms, as used herein, refers to clays which are themselves anionic in nature ie, the clays themselves are negatively charged at their surface and are capable of exchanging cations. Similarly, the term cationic clays and related terms, as used herein, refers to clays which are themselves cationic in nature ie, the clays themselves are positively charged at their surface and are capable of exchanging anions.

Preferred anionic clays are clays from the smectite class of clays. Typically, clays of this type are crystalline, expandable, three-layer clays.

Smectite clays are, for example, disclosed in US Patents Nos 3,862,058, 3,948,790, 3,954,632 and 4,062,647 and in EP-A-299,575 and EP-A-313,146, all in the name of Procter & Gamble Company.

The term smectite clays herein includes both the clays in which aluminium oxide is present in a silicate lattice and the clays in which magnesium oxide is present in a silicate lattice. Typical smectite clay compounds include the compounds having the general formula Al₂(Si₂O₅)₂(OH)₂.nH₂O and the compounds having the general formula Mg₃ (Si₂O₅)₂(OH)₂.nH₂O, and derivatives thereof, for example in which a proportion of the aluminium ions are replaced with magnesium ions or a proportion of the magnesium ions are replaced with lithium ions and/or some of the hydroxyl ions are replaced by fluoride ions; the derivatives may comprise a further metal ion to balance the overall charge. Smectite clays tend to adopt an expandable, three-layer structure.

Specific examples of suitable smectite clays include those selected from the classes of the montmorillonites, hectorites, volchonskoites, nontronites, saponites, beidelites and sauconites, particularly those having an alkali or alkaline earth metal ion within the crystal lattice structure. Particularly preferred are hectorites, montmorillonites, nontronites, saponites, beidelites, sauconites and mixtures thereof. Most preferred is synthetic hectorite.

Clays useful in the invention may be three-layer, expandable alumino-silicates which are characterised by a dioctahedral crystal lattice, while the expandable three-layer magnesium silicates have a trioctahedral crystal lattice. Other preferred clays have a mixed tetrahedral/octahedral/tetrahedral co-ordination.

The anionic clays employed in the compositions of the invention may contain cationic counterions such as protons, sodium ions, potassium ions, calcium ions, magnesium ions, and the like. It is customary to distinguish between clays on the basis of one cation predominantly or exclusively absorbed. For example, a sodium clay is one in which the absorbed cation is predominantly sodium. Such absorbed cations can become involved in exchange reactions with cations present in aqueous solutions. A typical exchange reaction involving a smectite-type clay is expressed by the following equation: smectite clay(Na) + NH₄OH → smectite clay(NH₄) + NaOH.

Since in the foregoing equilibrium reaction, one equivalent weight of ammonium ion replaces an equivalent weight of sodium, it is customary to measure cation exchange capacity (sometimes termed "base exchange capacity") in terms of milliequivalents per 100g of clay (meq/100g). The cation exchange capacity of clays can be measured in several ways, including by electrodialysis, by exchange with ammonium ion followed by titration or by a methylene blue procedure, all as fully set fourth in Grimshaw, "The Chemistry and Physics of Clays", pp. 264-265, Interscience (1971). The cation exchange capacity of a clay mineral relates to such factors as the expandable properties of the clay and the charge of the clay, which, in turn, is determined at least in part by the lattice structure, and the like.

Preferred anionic clays for use in the present invention have an ion exchange capacity of from 0.7meq/100g to 150meq/100g. Particularly preferred are clays having an ion exchange capacity of from 30 meq/100g to 100 meq/100g.

The clays preferably have an average particle size in the range of from 0.0001 µm to 800 µm, more preferably from 0.01 µm to 400 µm such as from 0.02 µm to 220 µm, even more preferably 0.02 µm to 100 µm. Particle sizes can be determined using a Malvern Mastersizer (Malvern Instruments, UK).

Examples of synthetic hectorites useful in the present invention include those products sold under the trade marks Laponite RD, Laponite RDS, Laponite XLG, Laponite XLS, Laponite D, Laponite DF, Laponite DS, Laponite S and Laponite JS (all from Southern Clay products, Texas, USA, a subsidiary of Rockwood).

Examples of montmorillonites (also known as bentonites) include: Gelwhite GP, Gelwhite H, Gelwhite L, Mineral Colloid BP, Mineral Colloid MO, Gelwhite MAS 100 (sc), Gelwhite MAS 101, Gelwhite MAS 102, Gelwhite MAS 103, Bentolite WH, Bentolite L10, Bentolite H, Bentolite L, Permont SX10A, Permont SC20, and Permont HN24 (Southern Clay Products, Texas, USA); Bentone EW and Bentone MA (Dow Corning); Bentonite USP BL 670 and Bentolite H4430 (Whitaker, Clarke & Daniels); Clarit 100 G1 and Clarit 1100 G1 (calcium bentonites from Süd Chemie AG); and Volclay 2 (sodium bentonite from Süd Chemie AG).

Organophilic clays may also be used herein. These are hydrophobically modified clays which have organic ions replacing inorganic metal ions by ion exchange processes known in the art. These kinds of clay are readily miscible with organic solvents and have the capability to absorb organic solvents at the interlayers. Suitable examples of organophilic clays useful in the invention are Bentone SD-1, SD-2 and SD-3 from Rheox of Highstown, NJ, USA.

Clays may be used in the present invention either singly or in combination with one or more other clays. If a mixture of clays is used, however, it is preferred that either all of the clays are anionic or all of the clays are cationic. However, mixtures of cationic and anionic clays may be used, provided that the dye remains sufficiently strongly bound to the clay mixture. Clays may be used as obtained from the supplier and may contain conventional additives such as, for example, disintegrating agents (also known as peptisers) and water of hydration.

Cationic clays suitable for use in the present invention preferably have physical properties (such as structure and particle size) similar to those of the anionic clays mentioned above. The clays are typically layered double hydroxides such as, for example, hydrotalcite. Suitable cationic clays are described in ,for example, US 5786381 (Franklin et al), the contents of which are incorporated by reference herein.

Preferred cationic clays for use in the present invention have an ion exchange capacity of from 0.7meq/100g to 250meq/100g. Particularly preferred are clays having an ion exchange capacity of from 30 meq/100g to 200 meq/100g.

Examples of cationic clays are the natural or synthetic layered double hydroxide clays. For instance, layered double hydroxides include compounds of formula [M(_{1- a)}Na(OH)₂}^{y+} X^{x-}_{y/x}.zH₂O where M is selected from divalent metal ions and lithium; N is a trivalent metal ion; X is an anion of charge x-; y+ is the net charge on the mixed metal hydroxide cation; and when M is a divalent metal ion, "a" is a number from 0.17 to 0.5 and y=a; and when M is lithium "a" is a number from 0.67 to 0.75 and y=(2a-1); and z is a number from 0 to 10. In these structures, the metal ions occur in layers in which the metal ions are connected together through the OH groups and the anions X, are located in interlayers between layers of metal ions. Furthermore, it is known that X can undergo ion exchange to be replaced by other anions eg organic anions. Various applications for these types of hydroxy materials have been described in the scientific literature, notably including their use as chemical catalysts.

Examples of cationic clays in the hydrotalcite category include Pural MG30, Pural MG50, Pural MG70 (from Condea Chemie GmbH, Hamburg, Germany). Hydrotalcites commercially available include Sorbacid EXM 911 and Hycite EM 713 (from Süd Chemie AG). Other examples of layered double hydroxides are Mg₄Al₂(OH)₁₂(SO₄)0.8. xH₂O Magaldrate (Guilini) and Mg₄Al₄(OH)₁₂Cl₂.xH₂O mixed metal hydroxide (Dow Chemicals). Bayerite is also a suitable cationic clay.

Compositions of the invention contain an agent which is capable of imparting a colour to hair. Such agents have a net positive or negative charge and are typically molecular cations or anions (ie, cations or anions comprising covalent bonds, including for example carbon-carbon bonds), preferably having a molecular weight in the range of from 200 Da to 3000 Da. The agent is normally used in the form of its salt with a charge-balancing counterion. For example, when the agent is positively charged, suitable anions include monovalent anions such as chloride and, when the agent is negatively charged, suitable cations include metal cations such as sodium ions.

The agent is preferably a cationic or anionic, semi-permanent dye.

Suitable anionic dyes for use in the present invention, which contain anionic agents, include azo dyes, xanthene dyes and dyes based on carbenium salts. Specific examples of dyes are:
6-hydroxy-5-[(4-sulfophenyl)azo]-2-naphthalenesulfonic acid disodium salt (CI 15985; Food Yellow No. 3); 2,4-dinitro-1-naphthol-7-sulfonic acid disodium salt (CI 10316; Acid Yellow No. 1; Food Yellow No. 1); 2-(2-quinolyl)-1H-indene-1,3(2H)-dione (mixture of mono- and disulfonic acid) (CI 47005; Food Yellow No. 13; Acid Yellow No. 3);
4,5-dihydro-5-oxo-1-(4-sulfophenyl)-4-[(4-sulfophenyl)azo]-1H-pyrazole-3-carboxylic acid trisodium salt (CI 19140; Food Yellow No. 4; Acid Yellow No. 23);
3',6'-dihydroxyspiro[isobenzofuran-1(3H),9'-[9H]xanthen]-3-one disodium salt (CI 45350; Acid Yellow No. 73; D &C Yellow No. 8);
5- [(2,4-dinitrophenyl)amino]-2-phenylaminobenzenesulfonic acid sodium salt (CI 10385; Acid Orange No. 3);
4-[(2,4- dihydroxyphenyl)azo]benzenesulfonic acid monosodium salt (CI 14270; Acid Orange No. 6);
4-[(2-hydroxy-1-naphthalenyl)azo]benzenesulfonic acid monosodium salt (CI 15510; Acid Orange No. 7);
4-[[3-[(2,4-dimethylphenyl)azo]-2,4-dihydroxyphenyl]azo]benzenesulfonic acid monosodium salt (CI 20170; Acid Orange No. 24);
4-hydroxy-3-[(4-sulfo-1-naphthalenyl)azo]-1-naphthalenesulfonic acid disodium salt (CI 14720; Acid Red No. 14);
7-hydroxy-8-[(4-sulfo-1-naphthalenyl)azo]-1,3-naphthalenedisulfonic acid trisodium salt (CI 16255; Ponceau 4R; Acid Red No. 18);
3-hydroxy-4-[(4- sulfo-1-naphthalenyl)azo]-2,7-naphthalenedisulfonic acid trisodium salt (CI 16185; Acid Red No. 27; Food Red 9);
5-amino-4-hydroxy-3-(phenylazo)-2,7-naphthalenedisulfonic acid disodium salt (CI 17200; Acid Red No. 33);
5-(acetylamino)-4-hydroxy-3-[(2-methylphenyl)azo]-2,7-naphthalenedisulfonic acid disodium salt (CI 18065; Acid Red No. 35);
3'-6'-dihyroxy-2',4',5',7'-tetraiodospiro[isobenzofuran-1(3H),9'-[9H]xanthen]-3-one disodium salt (CI 45430; Acid Red No. 51);
N-[6-(diethylamino)-9-(2,4-disulfophenyl)-3H-xanthen-3-ylidene] -N-ethylethaneamminium hydroxide, internal salt, sodium salt (CI 45100; Acid Red No. 52);
7-hydroxy-8-[[4-(phenylazo)phenyl]azo]-1,3-napthalenedisulfonic acid disodium salt (CI 27290; Acid Red No. 73);
2',4',5',7'-tetrabromo-3',6'-dihydroxyspiro[isobenzofurane-1(3H), 9'-[9H]-xanthen]-3-one disodium salt (CI 45380; Acid Red No. 87);
2',4',5',7'-tetrabromo-4,5,6,7-tetrachloro- 3', 6',-dihydroxyspiro[isobenzofuran-1(3H),9'-[9H]-xanthen]-3-one disodium salt (CI 45410; Acid Red No. 92);
3',6'-dihydroxy-4'5'- diiodospiro[isobenzofuran]-1(3H), 9'-(9H)-xanthen]-3-one disodium salt (CI 45425; Acid Red No. 95);
Benzenemethanaminium, N-ethyl-N-[4-[[4-[ethyl[(3-sulfophenyl)methyl]amino]phenyl](2-sulfophenyl)methylene]2,5-cyclohexadiene-1-ylidene]-3-sulpho-, hydroxide, inner salt disodium salt, CI 42090; Acid Blue No. 9);
2,2'-[(9,10-dihydro-9,19-dioxo-1,4-anthracenediyl)diimino]bis[5-methyl]benzenesulphonic acid disodium salt (CI 61570; Acid Green No. 25);
N-[4-[[4-(diethylamino)phenyl](2-hydroxy-3,6-disulfo-1-napthalenyl)methylene]-2,5-cyclohexadien-1-ylidene]-N-methylmethaminium hydroxide internal salt, monosodium salt (CI 44090; Food Green No. 4; Acid Green No. 50);
N-[4-[[4-(diethylamino)phenyl](2,4-disulfophenyl) methylene]-2,5-cyclohexadien-1-ylidene]-N-ethylethanaminium hydroxide internal salt, sodium salt (CI 42045; Food Blue No. 3; Acid Blue No. 1);
N-[4-[[4-(diethylamino)phenyl](5-hydroxy-2,4-disulfophenyl)methylene]2,5-cyclohexadien-1-ylidene]-N-ethylethanaminium hydroxide internal salt, calcium salt (2:1)(CI 42051; Acid Blue No. 3);
1-amino-4-(cyclohexylamino)-9, 10-dihydro-9, 10-dioxo-2-anthracenesulfonic acid monosodium salt (CI 62045; Acid Blue No.62);
2-(1,3-dihydro-3-oxo-5- sulfo-2H-indol-2-ylidene)-2, 3-dihydro-3-oxo-1H-indole-5-sulfonic acid disodium salt (CI 73015, Acid Blue No. 74);
9-(2-carboxyphenyl)-3-[(2-methylphenyl)amino]-6-[(2-methyl-4-sulfophenyl)amino]xanthylium hydroxide internal salt, monosodium salt (CI 45190; Acid Violet No. 9);
2-[(9.10-dihydro-4-hydroxy-9,10-dioxo-1-anthracenyl)amino]-5-methylbenzenesulfonic acid monosodium salt (CI 60730; D &C Violet No. 2; Acid Violet No. 43);
bis[3-nitro-4-[(4- phenylamino)-3-sulfophenylamino]phenyl]sulfone (CI 10410; Acid Brown No. 13);
4-amino-5-hydroxy-3-[(4-nitrophenyl)azo]-6-(phenylazo)-2,7- naphthalenedisulfonic acid disodium salt (CI 20470; Acid Black No. 1);
3-hydroxy-4-[(2-hydroxynaphth-1-yl)azo]-7-nitro-1-naphthalenesulfonic acid chromium complex (3:2) (CI 15711; Acid Black No. 52);
3-[(2,4-dimethyl-5-sulfophenyl)azo]-4-hydroxy-1-naphthalenesulfonic acid disodium salt (CI 14700; Food Red No. 1; Ponceau SX; FD &C Red No. 4);
4-(acetylamino)-5-hydroxy-6-[(7-sulfo-4-[(4-sulfophenyl]azo]-1-naphthalenyl)azo]-1,7-naphthalene disulfonic acid tetrasodium salt (CI 28440, Food Black No. 1); and
3-hydroxy-4-(3-methyl-5-oxo-1-phenyl-4, 5-dihydro- 1H-pyrazol-4-ylazo)naphthalene-1-sulfonic acid sodium salt, chromium complex (Acid Red No. 195).

Agents which are the anionic species in the so-called Acid dyes are particularly preferred.

Suitable cationic dyes for use in the present invention, which contain cationic agents, include:
3-[(4-amino-6-bromo-5,8-dihydro-1-hydroxy-8-imino-5-oxo-2-naphtyl)amino]-N,N,N-trimethylanilinium chloride (CI 56059; Basic Blue No. 99- Trade name Arianor Steel Blue & Jaracol Steel Blue);
mixture of: 8-[(4-amino-3-nitrophenyl)azo]-7-hydroxy-N,N,N-trimethyl-2-naphthaleneaminium chloride [Major];
8-[(4-amino-2-nitrophenyl)azo]-7-hydroxy-N,N,N-trimethyl-2-naphthaleneaminium chloride [Minor] (Basic Brown No. 17 - Arianor Sienna Brown & Jaracol Sienna Brown);
8-[(4-aminophenyl)azo]-7-hydroxy-N,N,N-trimethyl-2-naphthaleneaminium chloride(CI 12250; Basic Brown No. 16 - Arianor Mahogany & Jaracol Mahogany);
3-[4,5-dihydro-3-methyl-5-oxo-1-phenyl-1H-pyrazol-4-yl)azo]-N,N,N-trimethylanilinium chloride (CI 12719;Basic Yellow No. 57 - Arianor Straw Yellow & Jaracol Straw Yellow); and
7-Hydroxy-8-[(2-methoxyphenyl)azo]-N,N,N-trimethyl-2-naphthaleneaminium chloride (CI 12245; Basic Red No. 76 - Arianor Madder Red & Jaracol Madder Red).

The dyes mentioned above are available from Warner Jenkinson Europe, Kings Lynn, Norfolk, UK. Jaracol dyes are available from James Robinson Dyes, Huddersfield, UK.

Other examples of cationic dyes suitable containing cationic agents suitable for use in the invention include:
9-(dimethylamino)benzo[a]phenoxazin-7-ium chloride (CI 51175; Basic Blue No. 6);
di[4-(diethylamino)phenyl]-[4-(ethylamino)naphthyl]carbenium chloride (CI 42595; Basic Blue No. 7);
3,7-di-(dimethylaminophenothiazin-5-ium chloride (CI 52015; Basic Blue No. 9);
di[4-(dimethylamino)phenyl]-[4-(phenylamino)naphthyl]carbenium chloride (CI 44045; Basic Blue No. 26);
2[(4-(ethyl(2-hydroxyethyl)amino)phenyl)azo]-6-methoxy-3-methylbenzothiazolium methyl sulfate (CI 11154; Basic Blue No. 41);
bis[4-(dimethylamino)phenyl][4-(methylamino)phenyl]carbenium chloride (CI 42535; Basic Violet No. 1);
tris-[4-(dimethylamino)phenyl]carbenium chloride (CI 42555; Basic Violet No. 3);
2-[3,6-(diethylamino)dibenzopyranium-9-yl]benzoic acid chloride (CI 45170; Basic Violet No. 10);
di(4-aminophenyl)(4-amino-3-methylphenyl)carbenium chloride (CI 42510; Basic Violet No. 14);
1,3-bis[(2,4-diamino-5-methylphenyl)azo]-3-methylbenzene (CI 21010; Basic Brown No. 4);
1-[(4-amino-2-nitrophenyl)azo]-7-(trimethylammonio)-2-naphthol chloride (CI 12251; Basic Brown No. 17);
3,7-diamino-2,8-dimethyl-5-phenylphenazinium chloride (CI 50240; Basic Red No. 2);
1,4-dimethyl-5-[(4-(dimethylamino)phenyl)azo]-1,2,4-triazolium chloride (CI 11055; Basic Red No. 22);
2-[2-((2,4-dimethyoxyphenyl)amino)ethenyl]-1,3,3-trimethyl-3H-indol-1-ium chloride (CI 48055; Basic Yellow No. 11); and
bis[4-(diethylamino)phenyl)phenylcarbenium hydrogen sulfate (1:1) (CI 42040; Basic Green No. 1).

Agents which are the cationic species in the so-called Basic dyes are particularly preferred.

Agents capable of imparting a colour to hair may be used singly or as a mixture of two or more such agents. Preferably, when two or more agents are employed in compositions of the invention, either all of the agents are anionic or all of the agents are cationic. However, mixtures of anionic and cationic agents and mixtures of anionic and cationic clays may be employed in the invention.

In the compositions of the invention, the agent and the clay are selected such that they have opposite charges. Thus, cationic agents (for example, cationic dyes) are used together with anionic clays or anionic agents (for example, anionic dyes) are used together with cationic clays. Preferably, the compositions comprise an anionic clay and a cationic dye. Surprisingly, selecting and combining clays in this way provides the advantages of the invention, including low skin staining, whilst giving good delivery of colour to the hair.

Without wishing to be bound by theory, it is believed that the dye and the clay in the compositions of the invention may form some form of complex in which the dye and the clay are chemically and/or physically bound together, possibly as a result of the opposite charges on the two materials. The dye may be present at the surface of the clay particles and/or within the clay particles, such as by intercalation between layers in the clay particles.

The amounts of clay and agent are preferably selected such that, in the compositions of the invention, there is sufficient clay at least to balance the charge on the agent. However, this is not essential and a degree of binding of clay to the agent may be effected through polar but uncharged groups at the surface of the clay. Therefore, good results (including reduced skin staining) may be achieved with amounts of clay that are less than the amount that is required to balance the charge on the agent. Similarly, it is possible to use excess clay beyond the amount that balances the charge on the agent. The amount of clay and agent required in a charge-balanced composition can be readily determined; for example, the optimum level of agent and clay can be determined by mixing the agent and the clay (in water), removing the insoluble solids and determining spectrophotometrically the point at which the absorbance of the filtrate or supernatant liquor becomes a minimum with increasing clay content. Formulating the compositions in this way may help to optimise the balance between minimising skin staining and maximising colour delivery to the hair.

It is particularly preferred that the weight ratio of clay to agent is in the range of from 1:20 to 20:1, preferably 1:10 to 1:0.5. More preferably, the weight ratio of clay to agent is in the range of from 1:5 to 1:0.7, even more preferably from 1:4 to 1:0.9. The ratio of dye to clay used in any given case will depend on factors such as the molecular weight and charge of the dye and the surface area and charge on the clay.

The amount of clay in compositions of the invention is preferably in the range of from 0.01% to 20% by weight based on total composition, more preferably 0.1% to 10% by weight, most preferably 0.1% to 5% by weight.

The amount of the agent in compositions of the invention is preferably in the range of from 0.01% to 10% by weight based on total composition, more preferably 0.1% to 5% by weight, most preferably 0.1% to 2% by weight.

Compositions of the invention may comprise a single combination of a dye and a clay or a mixture of one or more dyes with one or more clays. Mixtures of two or more dye/clay complexes containing different dyes and the same or different clays have been found to be effective in varying the shade of colour imparted to hair.

Compositions of the invention comprise a perfume and/or a surfactant.

By the term perfume we mean any component which is added to the composition primarily for the purpose of imparting a fragrance to the composition (although the perfume may also have other functions and/or properties). Perfumes are conventionally added to compositions intended for application to the hair and are well-known to those skilled in the art. Perfumes may be single compounds or mixtures of two or more different compounds. A perfume will typically be present in the composition of the invention in an amount of from 0.001% to 5% by weight based on total weight of composition, more preferably 0.01% to 2% by weight, even more preferably 0.01% to 1% by weight.

Surfactants may also be present in compositions of the invention, typically in amounts of from 0.01% to 50% by weight based on total weight of composition, depending on the product form of the composition. Suitable surfactants include anionic, cationic, zwitterionic, amphoteric, non-ionic surfactants and mixtures thereof, and are described in more detail below in connection with specific product forms. The surfactant is selected for compatibility with the clay and agent of the invention and suitable surfactants can be readily identified by those skilled in the art.

Compositions of the invention are preferably produced by a method which comprises dispersing the clay in an aqueous liquid to form a dispersion and then bringing the agent into contact with the dispersion. The agent may be added to the dispersion of clay or the clay dispersion may be added to the agent. It has been found that such a method provides a composition which has good properties so far as colour delivery to hair and low skin staining are concerned. Preferably, in this method, the agent is in solid form when added to the dispersion eg, in the form of a powdered dye, although solutions of the dye may also be used. The dispersion may be stirred after the agent has been added (eg, for a time of 1 minute to 1 hour at from 10°C to 40°C). Typically, the clay is hydrated in the dispersion before the agent is added; this may require maintaining the clay in the dispersion for a sufficient time and at a sufficient temperature to allow hydration to take place; for example, the clay may be stirred in the aqueous liquid at a temperature of from 10°C to 40°C (preferably 10°C to 30°C) for a time of from 1 minute to 2 hours (preferably 10 minutes to 1 hour). The aqueous liquid may be water or water containing one or more other components (which are preferably water soluble) that are present in the final composition of the invention. Optionally, after the agent has been added to the dispersion, excess aqueous liquid (eg, water) may be removed: for example, by filtration or by centrifuging and withdrawing the supernatant liquor.

The invention provides a method of colouring hair which comprises applying to the hair a composition of the invention. The method may be primarily or solely intended for colouring the hair, in which case the composition will be a hair colourant composition. Alternatively, the method may achieve one or more other functions besides colouring hair. For example, the composition may be a hair conditioning composition further comprising a hair conditioning agent, in which case the method may also effect conditioning of the hair. Similarly, the composition may be a shampoo composition further comprising a surfactant, in which case the method may also effect cleaning of the hair; shampoo compositions may also condition the hair (eg, if they are so-called 2-in-1 compositions) and, therefore, compositions of the invention may both condition and clean the hair, in addition to colouring the hair.

When the composition is applied to the hair, the agent and the clay are applied to the hair at the same time ie, simultaneously and in one step. The agent and the clay may be in the form of a preformed complex.

Compositions of the invention may be used to colour hair in conventional ways eg, by application of the composition to the hair in a specific hair colouring step which may, for example, be employed every two to eight weeks. Preferably, however, the compositions are applied to hair on a regular basis from a rinse-off hair treatment product, for example from twice daily to once every week, as part of a hair cleaning (ie, shampooing) and/or conditioning treatment.

Compositions of the invention typically contain water, preferably in an amount of from 50% to 99% by weight based on total composition, preferably 50% to 95% by weight, more preferably 75% to 95% by weight. Other solvents, for example alcohols containing from one to four carbon atoms (such as ethanol) may also be present in compositions of the invention.

The method of the invention may be applied to any type of hair. For example, the method may be applied to pigmented or unpigmented hair eg, white or grey hair. The method may also be used to colour hair which is naturally pigmented or otherwise coloured (eg, by an earlier dyeing step). The method may involve the use of implements such as brushes, applicators, pump action aerosol sprays, combs and the like. It has been found that frictional forces, particularly shear forces, can enhance colour delivery from compositions of the invention, especially from rinse off products.

Besides the actives, the compositions of the present invention may also contain other ingredients conventionally used in the art such as diluents, sequestrants, thickeners, carriers, surfactants (anionic, cationic, nonionic, amphoteric, zwitterionic and mixtures thereof), antioxidants, proteins, polypeptides, preservatives, moisturising agents, solvents, perfumes, enzymes, polymers and conditioners. Compositions of the invention are rinse off products. Rinse off compositions are compositions that are intended to be rinsed from the hair, typically with water, after use. Most, but not all, of the composition is removed from the hair on rinsing. The compositions are normally rinsed off the hair within 1 hour of application, typically within 30 minutes of application to the hair. Rinse off compositions include shampoos, conditioners and hair colourant compositions.

### Shampoo and/or Conditioner Compositions

Shampoo and/or conditioner compositions are preferred product forms for compositions of the invention, although the compositions may also take other product forms.

Shampoo compositions of the invention comprise at least one surfactant which provides a deterging benefit. The deterging surfactant is preferably selected from anionic, nonionic, amphoteric and zwitterionic surfactants, and mixtures thereof.

Suitable anionic surfactants include the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium ammonium and mono-, di- and triethanolamine salts.

The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

Nonionic surfactants suitable for use in compositions of the invention may include condensation products of aliphatic (C₈-C₁₈) primary or secondary linear or branched chain alcohols or pnenols with alkylene oxides, usually ethylene oxide and generally having from 6 to 30 ethylene oxide groups. Other suitable nonionics include mono- or di-alkyl alkanolamides. Example include coco mono- or di- ethanolamide and coco mono-isopropanolamide.

Further nonionic surfactants which can be included in shampoos for the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group. Suitable alkyl polyglycosides for use in the invention are commercially available and include for example those materials identified as: Oramix NS10 ex Seppic; Plantaren 1200 and Plantaren 2000 ex Henkel.

Amphoteric and zwitterionic surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates alkyl amidopropyl hydroxysultaines, acyl taurates and acyl glutamates, wherein the alkyl and acyl groups have from 8 to 19 carbon atoms. Examples include lauryl amine oxide, cocodimethyl sulphopropyl betaine and preferably lauryl betaine, cocamidopropyl betaine and sodium cocamphopropionate.

The surfactants are present in shampoo compositions of the invention in an amount of from 0.1 to 50% by weight of the composition, preferably from 0.5 to 30% by weight.

Compositions in accordance with the invention may also take the form of hair conditioning compositions, which are rinse off hair conditioning compositions or so-called 2 in 1 compositions containing shampoo and conditioner. The conditioning compositions preferably comprise one or more cationic surfactants. The use of cationic surfactants is especially preferred, because these ingredients are capable of providing conditioning benefits to hair.

Examples of cationic surfactants include: quaternary ammonium hydroxides, e.g., tetramethylammonium hydroxide, alkyltrimethylammonium hydroxides wherein the alkyl group has from about 8 to 22 carbon atoms, for example octyltrimethylammonium hydroxide, dodecyltrimethy- ammonium hydroxide, hexadecyltrimethylammonium hydroxide, cetyltrimethylammonium hydroxide, octyldimethylbenzylammonium hydroxide, decyldimethylbenzylammonium hydroxide, stearyldimethylbenzylammonium hydroxide, didodecyldimethylammonium hydroxide, dioctadecyldimethylammonium hydroxide, tallow trimethylammonium hydroxide, cocotrimethylammonium hydroxide, and the corresponding salts thereof, e.g., chlorides, Cetylpyridinium hydroxide or salts thereof, e.g., chloride ,Quaternium -5, Quaternium - 31,Quaternium -18, and mixtures thereof.

In hair conditioning compositions according to the invention, the level of cationic surfactant is preferably from 0.01 to 10%, more preferably 0.05 to 5%, most preferably 0.1 to 2% by weight of the composition.

Hair conditioning compositions of the invention may also contain one or more conditioning agents, preferably selected from silicones, protein hydrolysates and quaternised protein hydrolysates and other materials which are known in the art as having desirable hair conditioning properties.

Silicones are the most preferred conditioning agents.

Suitable silicones include volatile and non-volatile silicones, such as for example polyalkylsiloxanes, polyalkylaryl siloxanes, siloxane gums and resins, cyclomethicones, aminofunctional silicones, quaternary silicones and mixtures thereof. Silicone oil is a particularly preferred conditioning agent for hair. The silicone may be in the form of a low viscosity oil which may contain a high viscosity oil or gum in solution. Alternatively, the high viscosity material may be in the form of an emulsion in water. The emulsion may be of high viscosity oil or of a solution of gum in a lower viscosity oil. The particle size of the oil phase may be anywhere in the range from 30 nanometres to up to 20 microns average size.

The silicone oil may suitably be a polydimethylsiloxane with an average particle size of less than 20 microns and preferably less than 2 microns. Small particle size enables a more uniform distribution of silicone conditioning agent for the same concentration of silicone in the composition. Advantageously, a silicone with a viscosity in the range 1-20 million cst is used. The silicone can be cross-linked.

Preferred silicones include polydimethylsiloxanes (of CTFA designation dimethicone) and hydroxylated polydimethylsiloxanes (of CTFA designation dimethiconol). Silicones of the above types are widely available commercially, for example as DC-1784 and DCX2-1391, both ex Dow Corning.

Suitable protein hydrolysates include lauryl dimonium hydroxy propylamino hydrolysed animal protein, available commercially under the trade name LAMEQUAT L, and hydrolysed keratin containing sulphur-bearing amino acids, available commercially under the trade name CROQUAT WKP.

In accordance with the invention, the hair shampoo and/or conditioner composition may also comprise a polymeric water-soluble cationic polymer as a conditioning agent.

The cationic polymer may be present at levels of from 0.01 to 5%, preferably from about 0.05 to 1%, more preferably from about 0.08% to about 0.5% by weight.

Synthetic or naturally derived polymers having a quaternised nitrogen atom are useful. The molecular weight of the polymer will generally be between 5,000 and 10,000,000 Da, typically at least 10,000 Da and preferably in the range 100,000 to about 2,000,000 Da.

Representative synthetic quaternised polymers include, for example: cationic copolymers of 1-vinyl-2-pyrrolidine and 1-vinyl-3-methyl-imidazolium salt (e.g., Chloride salt) (referred to in the industry by the Cosmetic, Toiletry, and Fragrance Association, "CTFA". as Polyquaternium-16); copolymers of 1-vinyl-2-pyrrolidine and dimethylaminoethyl methacrylate (referred to in the industry by CTFA as Polyquaternium-11); cationic diallyl quaternary ammonium-containing polymers including, for example, dimethyldiallylammonium chloride homopolymer (referred to in the industry (CTFA) as Polyquaternium 6); mineral acid salts of amino-alkyl esters of homo-and co-polymers of unsaturated carboxylic acids having from 3 to 5 carbon atoms, as described in U.S. Patent 4,009,256; and cationic polyacrylamides as described in WO95/22311.

Representative naturally-derived quaternised polymers include quaternised cellulosic compounds and cationic guar gum derivatives, such as guar hydroxypropyltrimonium chloride. Examples are JAGUAR C-13S, JAGUAR C-15, and JAGUAR-C17, commercially available from Meyhall in their JAGUAR (trademark) series.

Suitable cationic polyacrylamides are described in WO 95/22311 whose contents are incorporated herein by reference.

The compositions may further comprise from 0.1 to 5 % by weight of a suspending agent. Examples are polyacrylic acids, cross linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearates, alkanolamides of fatty acids having from 16 to 22 carbon atoms and mixtures thereof. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol materials are available from Goodrich and Carbopol is a trade mark. A further suitable suspending agent is dihydrogenated tallow phthalic acid amide (available from Stepan under the trademark Stepan TAB-2).

Suitable cross linked polymers of acrylic acid and acrylate esters are Pemulen TR1 or Pemulen TR2. A suitable heteropolysaccharide gum is xanthan gum, for example that available as Kelzan mu.

Another ingredient that may advantageously be incorporated into shampoo and/or conditioning compositions of the invention is a fatty alcohol material. The use of these materials is especially preferred in conditioning compositions of the invention, in particular conditioning compositions which comprise one or more cationic surfactant materials. The combined use of fatty alcohol materials and cationic surfactants in conditioning compositions is believed to be especially advantageous, because this leads to the formation of a lamellar phase, wherein the cationic surfactant is dispersed.

Preferred fatty alcohols comprise from 8 to 22 carbon atoms, more preferably 16 to 20. Examples of preferred fatty alcohols are cetyl alcohol and stearyl alcohol. The use of these materials is also advantageous in that they contribute to the overall conditioning properties of compositions of the invention.

The level of fatty alcohol materials is conveniently from 0.01 to 10%, preferably from 0.1 to 5% by weight of the composition. The weight ratio of cationic surfactant to fatty alcohol is preferably from 10:1 to 1:10, more preferably from 4:1 to 1:8, most preferably from 1:1 to 1:6.

A further ingredient that may be desirably included in the shampoo and/or conditioning compositions is a pearlescent material. Suitable pearlescent materials include ethylene glycol distearate, ethylene glycol monostearate, guanine and titanium dioxide coated micas, bismith oxychloride, and stearic monoethanol amide. The level of pearlescent material present in the composition is generally 0.1% to 5%, preferably from 0.3% to 3% by weight of the composition.

The shampoo and/or conditioner compositions of the invention are preferably aqueous based. The compositions suitably comprise water in amount of from about 20 to about 99% by weight of the total composition.

The invention will now be described, by way of nonlimiting example only, with reference to the following examples. In the examples and throughout this specification, all references to percentages are to percentages by weight unless indicated otherwise.

### EXAMPLES

### Examples 1 to 9

Laponite XLS (3% w/w) was added to water (96%) and stirred with an impeller at 300 rpm for 30 minutes until the solution became clear. 1% w/w Arianor Mahogany dye powder (Warner Jenkinson, Norfolk, UK) was added and stirred for a further 30 minutes. The resulting dye/clay complex was concentrated by removing excess water by filtration under vacuum or by centrifuging and withdrawing the supernatant liquor.

The following are examples of hair conditioner compositions containing the dye/clay complex thus prepared.

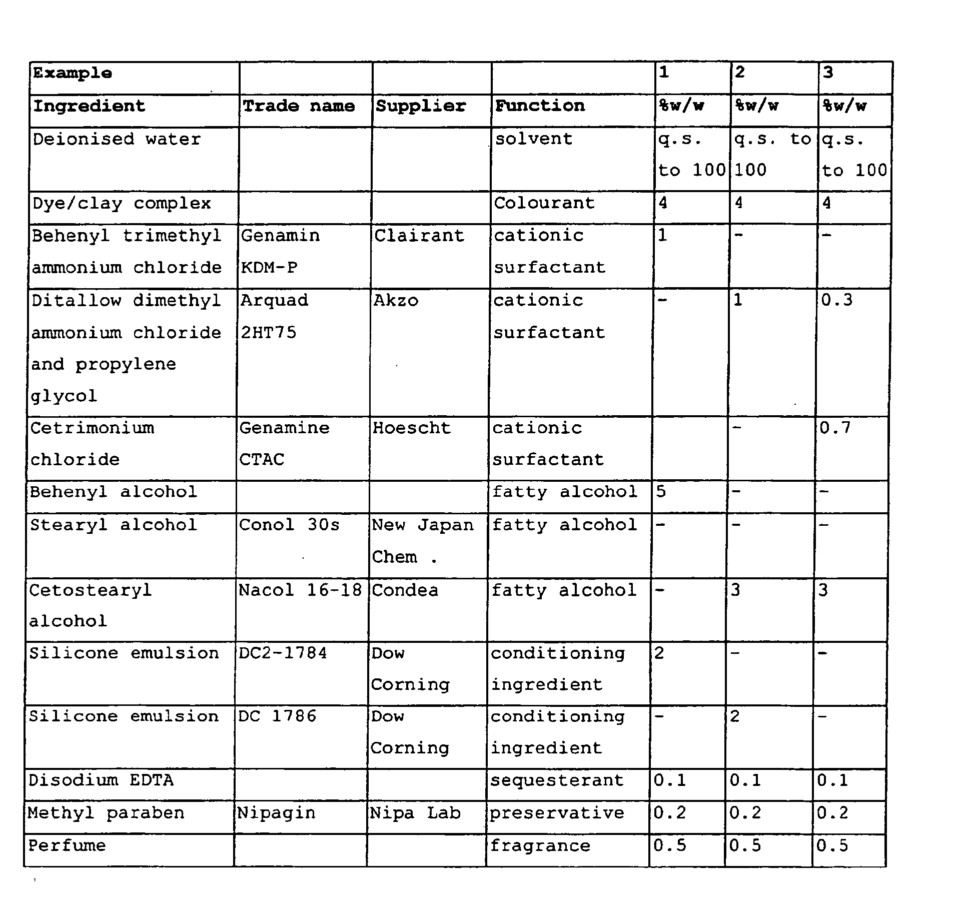

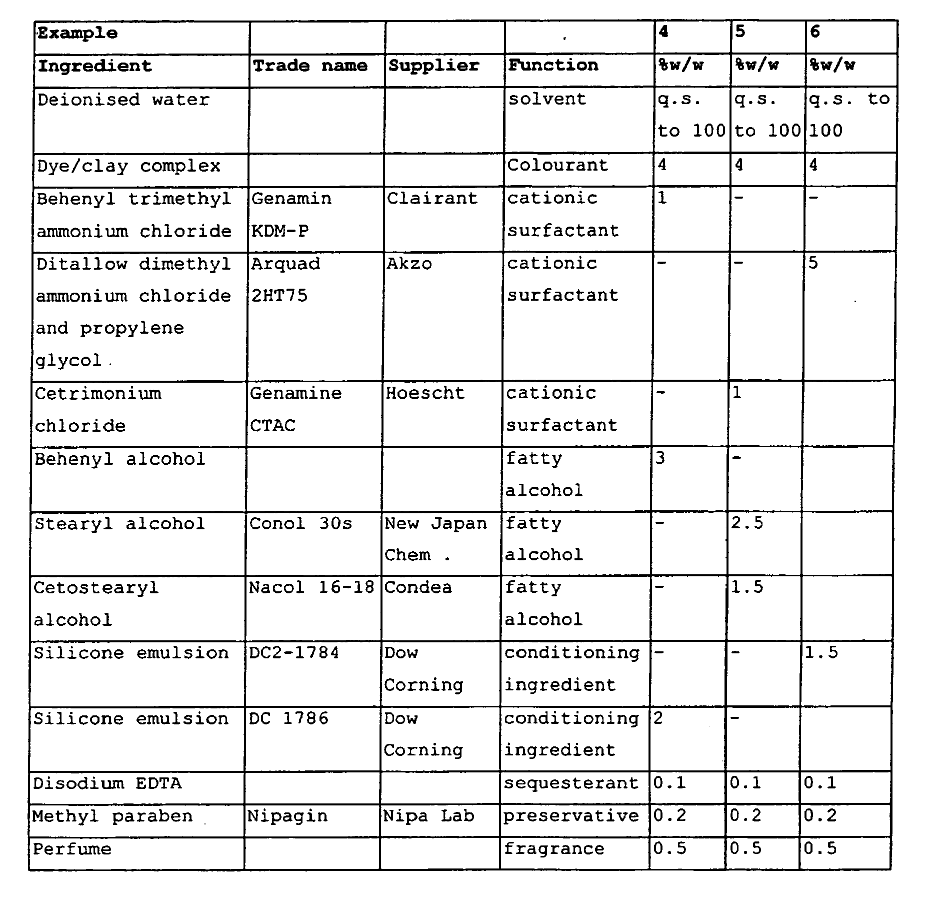

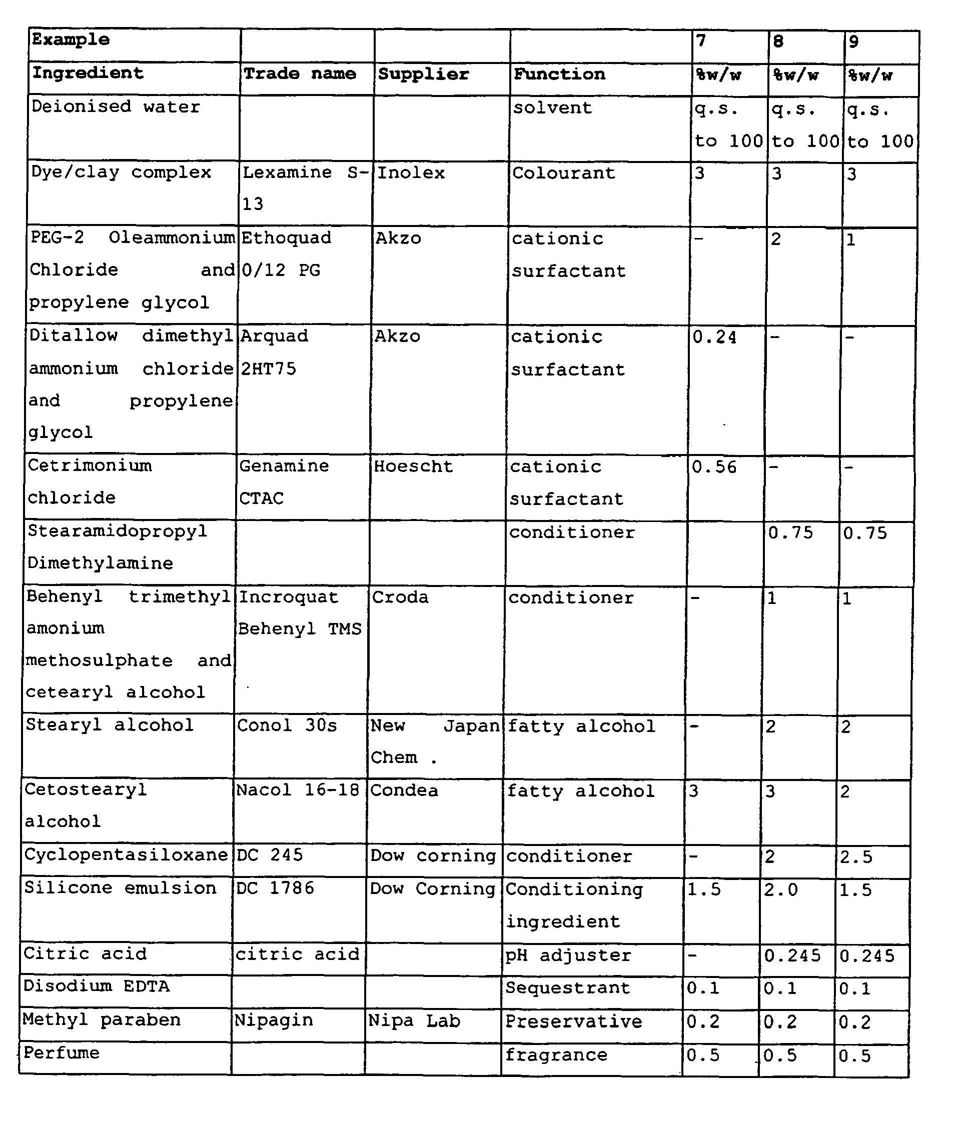

The compositions of Examples 1 to 9 can be produced as follows.

The cationic surfactant is dissolved in water at 90°C. The fatty alcohols are pre-melted at about 60°C. The molten fatty alcohols are added to the cationic surfactant solution at 70°C and a shear force is applied (Silverson L4R) for 30 minutes. The mixture is cooled slowly and the dye/clay complex, the silicone emulsion, perfume and ancilliary ingredients are added at a temperature below 45°C.

### Example 10

Mixtures of Mahogany dye, Laponite XLS and water were prepared according to the method described above. Thus, Laponite XLS was added to water and stirred with an impeller at 300 rpm for 30 minutes until the solution became clear and the Mahogany dye powder was added and stirred for a further 30 minutes. Compositions containing 1% by weight dye, various amounts of Laponite XLS and balance water were prepared.

The compositions were assessed for their colour delivery by application of 0.15g of composition for 60 sseconds to switches (also known as tresses or swatches) of yak hair (International Hair Importers and Products, Inc, NY, USA), followed by rinsing with water for 60 s and analysis of the treated hair by reflectometer (ColorQuest, HunterLab ,VA, USA) and the following results were obtained:

| % w/w Laponite XLS | Change in colour (ΔE) |
|---|---|
| 0 | 25 |
| 0.5 | 27 |
| 1.0 | 32 |
| 2.5 | 39 |
| 3.0 | 41 |
| 5.0 | 38 |
| 10 | 18 |
| 12.5 | 23 |
| 15 | 20 |

Thus, good colour delivery to the hair is seen throughout the compositions tested, with surprisingly good delivery at lower levels of Laponite XLS beyond the levels achieved by the use of the dye alone.

The same compositions were assessed for their skin staining properties in vitro by application of 0.25 g of composition for 60 s, rubbed in for 30 s and left to stand for 30 s, followed by rinsing in 1 litre of water for 60 s, to samples of skin obtained from IMS Inc, Milford CT, USA. The change in colour was again determined using a reflectometer.

| % w/w Laponite XLS | Change in colour (ΔE) |
|---|---|
| 0.5 | 64 |
| 1.0 | 61 |
| 2.5 | 11 |
| 3.0 | 12 |
| 5.0 | 8 |
| 10 | 2 |
| 12.5 | 2 |
| 15 | 2 |

Thus, skin staining was greatly reduced for compositions containing the clay in an amount of 2.5% by weight and above, whilst, as shown above, the levels of hair colouration were maintained.

### Example 11

A dye/clay complex containing Mahogany dye and Laponite XLS clay at a weight ratio of 1 dye: 2.5 clay was prepared according to the procedure described above.

The dye/clay complex was formulated as a 0.2% by weight dye/ 0.5% by weight clay hair conditioning composition in water also comprising 1% by weight behenyl trimethylammonium chloride (BTAC) and 6% by weight behenyl alcohol or 1% by weight ditallow dimethyl ammonium chloride (Diquat) and 3% by weight cetostearyl alcohol according to the procedure described above. The compositions were applied to switches of yak hair and the change in colour was determined using a reflectometer. The change in colour was compared to compositions containing the same level of dye and dye/clay complex alone (in water) after rinsing the product from the hair. The results are as follows:

| Treatment | Change in colour (ΔE) |
|---|---|
| 0.2% Mahogany dye solution | 19.8 |
| 0.2% Dye/0.5% clay complex alone | 24.6 |
| Dye/clay complex with 1% BTAC /6% behenyl alcohol | 15.9 |
| Dye/clay complex with 1% Diquat /3% cetostearyl alcohol | 15.9 |

Thus, the dye/clay complex can be effectively delivered to the hair from a rinse-off daily hair treatment product.

The same complexes and compositions of the invention showed a reduction in skin staining as shown below.

| Treatment | Skin Staining (ΔE) |
|---|---|
| 0.2% Mahogany dye solution | 58.1 |
| 0.2% Dye/0.5% clay complex alone | 10.1 |
| Dye/clay complex with 1% BTAC /6% behenyl alcohol | 8.4 |
| Dye/clay complex with 1% Diquat /3% cetostearyl alcohol | 9.1 |

### Example 12

Example 3 was repeated using Mahogany dye complexed with bentonite at a weight ratio of dye to clay of 1:3. The results of colour delivery on hair and skin staining were as follows.

| Treatment | Change in colour(ΔE) |
|---|---|
| 1% wt Mahogany dye | 20.8 |
| 1% dye/ 3% clay complex alone | 16.1 |
| Dye/clay complex in Example 3 | 10.3 |

The same complexes and compositions of the invention showed a reduction in skin staining as shown below.

| Treatment | Skin Staining(ΔE) |
|---|---|
| 1% wt Mahogany dye | 65.4 |
| 1% dye/ 3% clay complex alone | 21.5 |
| Dye/clay complex in Example 3 | 8.3 |

### Example 13

Example 12 was repeated using Bentone EW, an organically modified clay, in place of bentonite. Compositions were formulated as 2:1 and 1:1 by weight clay to dye dispersions in water at 3% complex by weight and 2% complex by weight, respectively. The 1:1 composition was as good as the corresponding composition containing Laponite XLS in place of Bentone EW at delivering colour to the hair.

### Examples 14 to 23

Anionic dye/cationic clay complexes were prepared according to Examples 26-29. Anionic clay/cationic dye complexes were prepared according to Examples 11 and 12. The following are examples of shampoo compositions incorporating the previously mentioned dye clay complexes according to the invention:

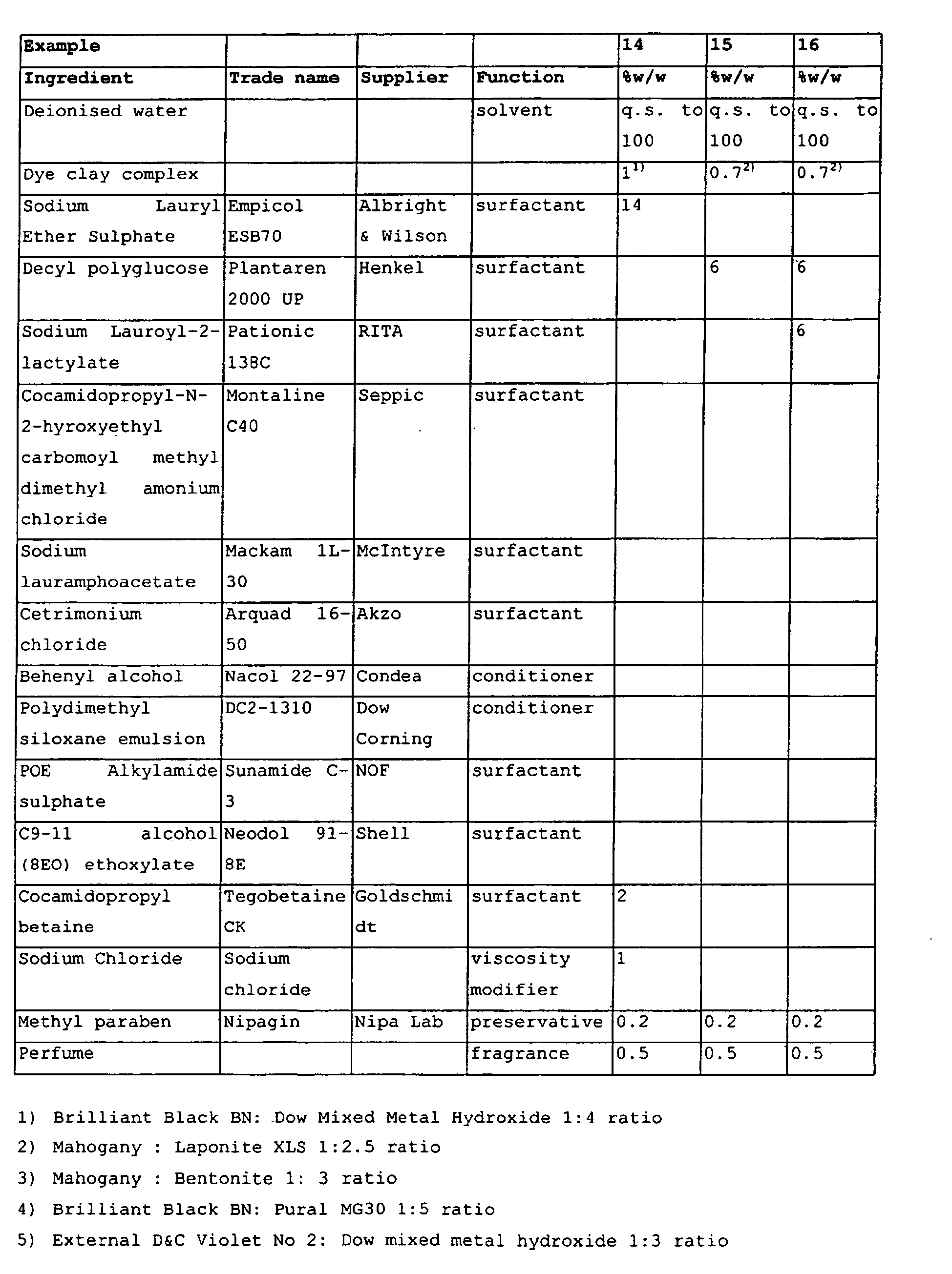

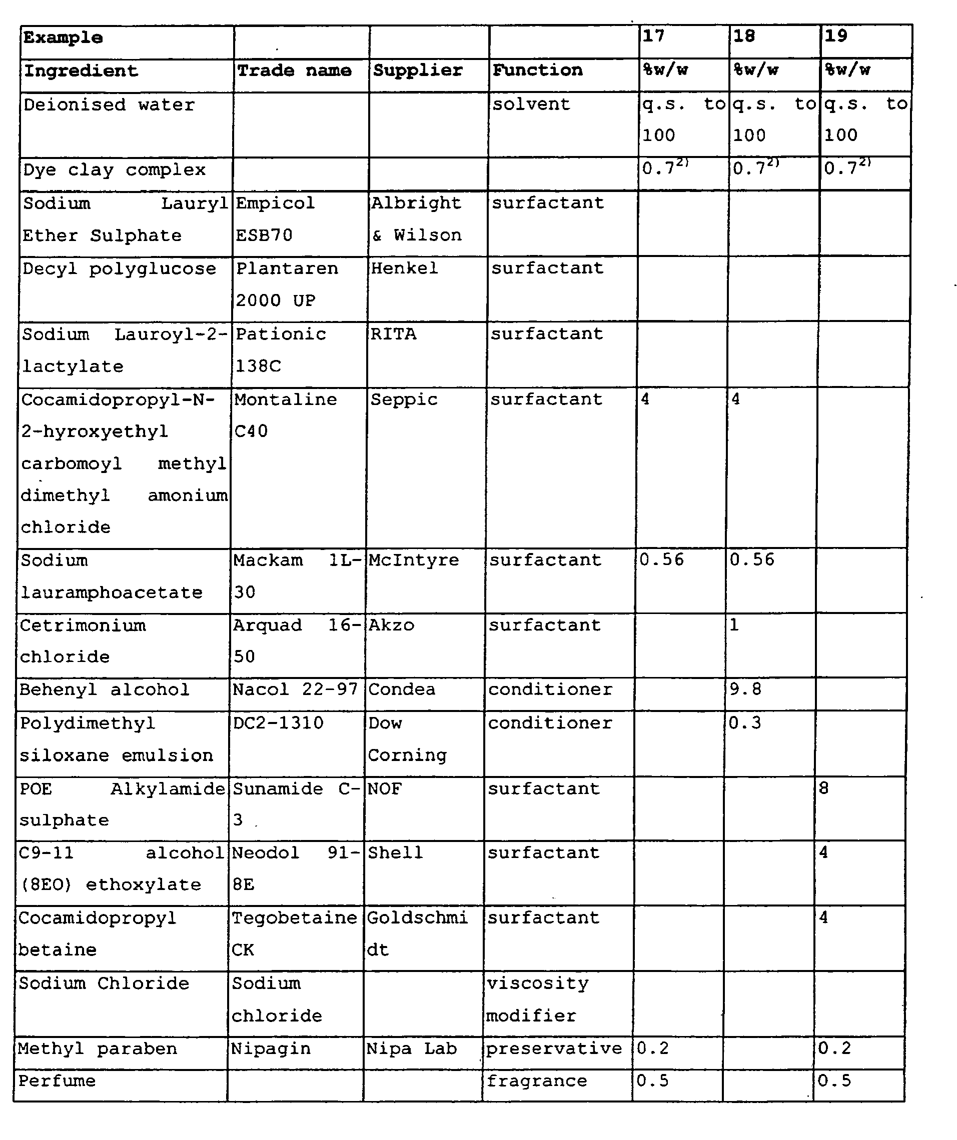

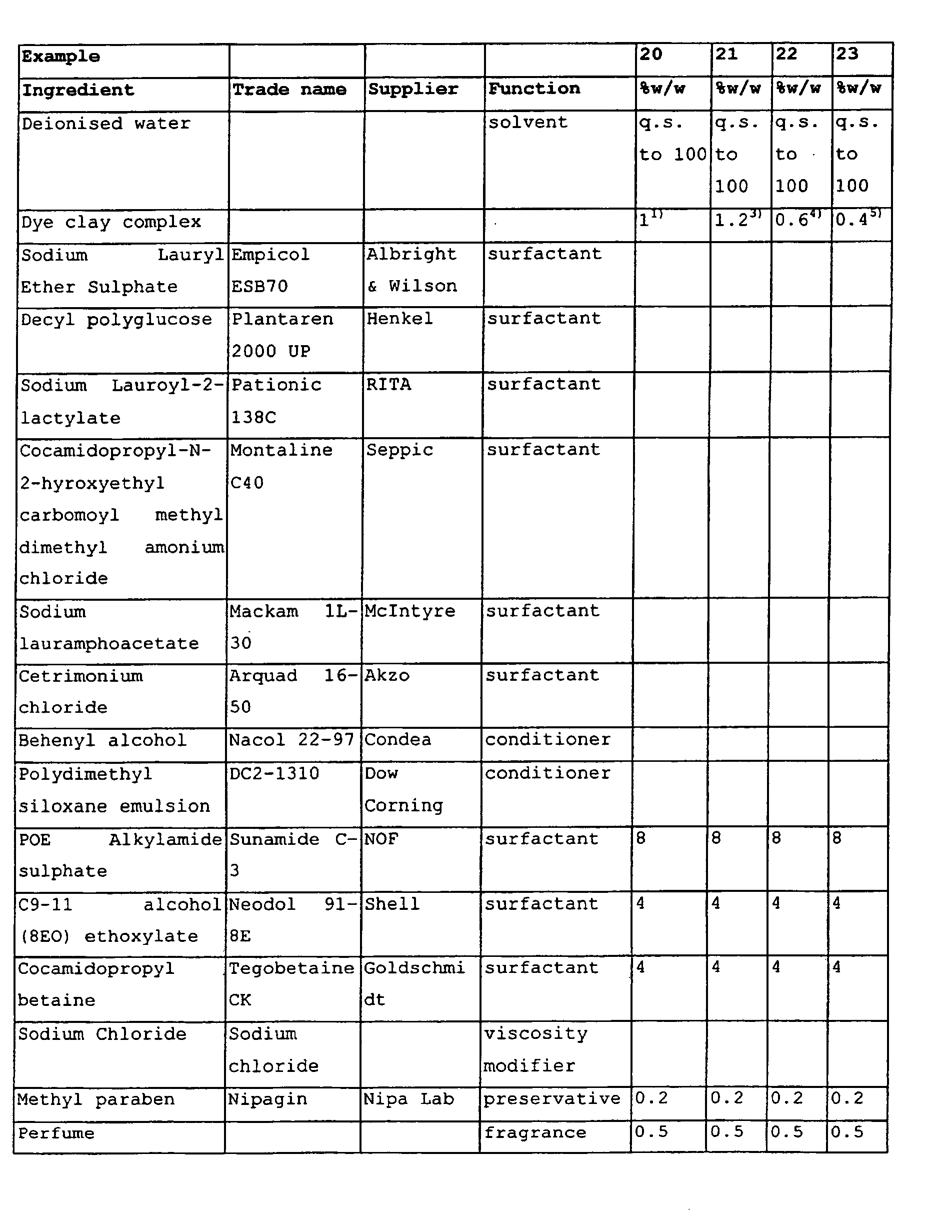

The shampoo formulations were tested for colour delivery by treating human natural white with 0.15 g of composition rubbed through a hair switch for 60 s, followed by rinsing with water for 60 s. The compositions showed colour delivery onto hair as follows:

| Treatment | Change in colour(ΔE) |
|---|---|
| Example 14 | 2.8 |
| Example 15 | 9.0 |
| Example 16 | 5.9 |
| Example 17 | 11.9 |
| Example 18 | 15.2 |
| Example 19 | 2.1 |
| Example 20 | 6.8 |
| Example 21 | 3.7 |
| Example 22 | 5.1 |
| Example 23 | 4.0 |

The same complexes and compositions of the invention showed a reduction in skin staining compared to the same composition with an equal amount of free dye.

Furthermore, Example 17 showed a build up of colour over several wash cycles as shown below:

| Treatment | Change in colour (ΔE) |
|---|---|
| 1 Wash cycle with Example 17 | 11.9 |
| 2 Wash cycles with Example 17 | 16.4 |
| 3 Wash cycles with Example 17 | 19.0 |
| 4 Wash cycles with Example 17 | 21.9 |

### Example 24

Example 3 was repeated using human natural white hair and a dye/clay complex was prepared using 1g Madder Red dye for every 2g Laponite XLS. Colour delivery from the hair conditioning composition onto hair was of the same order as delivery from an equal concentration of the dye in the hair conditioning composition. Reduced skin staining was observed when the dye/clay complex was incorporated in a hair conditioning composition compared to the same amount of free dye in the hair conditioning composition as shown below:

| Treatment | ΔE) Change in colour | (ΔE) Skin Staining |
|---|---|---|
| 0.8% Madder Red | 24.2 | 26.3 |
| 0.8% Madder Red/clay complex | 21.5 | 10.7 |

Madder Red dye is a dye of limited solubility. Using the dye in the form of a dye/clay complex according to the invention allowed a greater amount of dye to be used in the compositions ie, the amount of dye that could be used was no longer limited by the solubility of the dye in water.

| Treatment | Change in colour (ΔE) |
|---|---|
| 0.8% Madder Red in water | 23.7 |
| 0.8% Madder Red/clay complex | 40.8 |

### Example 25

Example 24 was repeated using anionic hectorite clay containing fluoride ions (Laponite JS) and Steel Blue dye at a dye to clay weight ratio of 1:2. The complex showed delivery of colour to the hair from a conditioner composition, with reduced skin staining compared to the uncomplexed dye in an otherwise identical hair conditioning composition as shown below.

| | |
|---|---|
| Treatment | Change in colour (ΔE) |
| 0.2% w/w Steel Blue in Example 8 | 11.5 |
| 0.2% w/w Steel Blue/clay complex in Example 8 | 11.1 |
| 1% w/w Steel Blue Solution | 25.0 |
| 1% w/w Steel Blue/clay complex | 26.3 |

| Treatment | (ΔE) Skin Staining |
|---|---|
| 1% w/w Steel Blue in Example 8 | 34.4 |
| 1% w/w Steel Blue/clay complex in Example 8 | 12.0 |

### Example 26

Complexes containing cationic clays were prepared as follows.

2.79g of Pural MG30 was added to 46.71g of water in a 100 ml beaker. 0.5g of dye (either Brilliant Black BN or Ext D&C Violet No. 5) was then added and the mixture was homogenised for 30 mins at 24,000 rpm using an UltraTurax mixer (IKA) fitted with a T25 dispersing tool. The resulting 1:5.5 weight ratio dye:clay mixture was then added to the hair conditioning composition base in the manner previously described.

The complexes were applied to hair from a hair conditioning composition containing a cationic hair conditioning agent. Colour delivery to human natural white hair is shown below:

| Treatment (% by wt) | (ΔE) Change in colour | (ΔE)Skin Staining |
|---|---|---|
| 0.1% Ext D&C Violet No. 2 in Example 2 | 8.6 | - |
| 0.1% Ext D&C Violet No. 2/ clay complex in Example 2 | 18.0 | - |
| 0.1% Brilliant Black BN in Example 2 | 25.1 | 64.4 |
| 0.1% Brilliant Black BN/ | | |
| clay complex in Example 2 | 21.4 | 4.8 |

Thus the hair conditioner composition containing the clay complex with Ext D&C Violet No. 2 showed better colour delivery than the corresponding conditioner with free dye. The colour delivery for the conditioner containing Brilliant Black BN was slightly higher than that shown by the Brilliant Black BN complex. However, the conditioner composition containing Brilliant Black BN dye showed reduced stability in terms of its structure and produced much higher skin staining.

### Example 27

2.0g of Layered double hydroxide (Dow Mixed metal hydroxide) was added to 47.5g of water in a 100 ml beaker. 0.5g of Brilliant Black BN was then added and the mixture was homogenised for 5 mins at 11,000 rpm using an UltraTurax mixer (IKA) fitted with a T25 dispersing tool. The resulting 1:4 weight ratio dye:clay mixture was then added to the conditioner base in the manner previously described.

The dye/clay complex alone or once incorporated into Example 3 showed good colour delivery to human natural white hair as illustrated below:

| Treatment (% by wt) | Change in colour (ΔE) |
|---|---|
| 0.2% Brilliant Black BN in water | 3.3 |
| 0.2% Brilliant Black BN/clay complex only | 28.6 |
| 0.2% Brilliant Black BN/clay complex in Example 3 | 10.8 (2.4 skin staining) |

### Example 28

Example 27 was repeated using Magaldrate (Giulini, Corp NJ, USA) as the clay.

### Example 29

1.5g of Layered double hydroxide was added to 48g of water in a 100 ml beaker. 0.5g of Ext D&C Violet No 2 was then added and the mixture was homogenised for 5 mins at 11,000 rpm using an UltraTurax mixer (IKA) fitted with a T25 dispersing tool. The resulting 1:3 weight ratio dye:clay mixture was then added to the conditioner base in the manner previously described.

The dye/clay complex alone or once incorporated into a formulation according to Example 3 (but with the different dye/clay complex) showed good colour delivery to human natural white hair as illustrated below:

| Treatment (% by weight) | Change in colour (ΔE) |
|---|---|
| 0.2% Ext D&C Violet No 2 in water | 4.3 |
| 0.2% Ext D&C Violet No 2/clay complex only | 22.4 |
| 0.2% Ext D&C Violet No 2/clay complex in Example 3 | 8.4 |

## Claims

1. A method of colouring hair comprising the step of applying to the hair a rinse off composition comprising:
(i) a clay having a net positive or negative charge at its surface;
(ii) an agent capable of imparting a colour to hair; and
(iii) a perfume and/or a surfactant,
wherein the agent has a net charge which is opposite from the charge on the surface of the clay.

2. A method as claimed in Claim 1, wherein the clay has a layered structure.

3. A method as claimed in Claim 1 or Claim 2 wherein the clay is present in the form of a dispersion or suspension of clay particles.

4. A method as claimed in any one of the preceding claims, which contains at least sufficient clay to balance the charge on the agent.

5. A method as claimed in any one of the preceding claims, wherein the weight ratio of clay to agent is in the range of from 1:10 to 1:0.5.

6. A method as claimed in Claim 5, wherein the weight ratio of clay to agent is in the range of from 1:5 to 1:0.7.

7. A method as claimed in any one of the preceding claims, wherein the agent is a molecular cation or a molecular anion.

8. A method as claimed in any one of the preceding claims, wherein the clay is an anionic clay and the agent is a cationic dye.

9. A method as claimed in Claim 8, wherein the clay is from the smectite class of clays.

10. A method as claimed in Claim 9, wherein the clay is a natural, synthetic or chemically modified clay of the type selected from the group consisting of hectorite, montmorillonite, nontronite, saponite, beidelite, sauconite and mixtures thereof.

11. A method as claimed in Claim 10, wherein the clay is synthetic hectorite.

12. A method as claimed in any one of Claims 8 to 11, wherein the agent is the cation of a Basic dye

13. A method as claimed in any one of Claims 1 to 7, wherein the clay is a cationic clay and the agent is an anionic dye.

14. A method as claimed in Claim 13, wherein the clay is a natural or synthetic layered double hydroxide clay.

15. A method as claimed in Claim 13 or Claim 14, wherein the agent is the anion of an Acid dye.

16. A method as claimed in any one of the preceding claims wherein the composition is a hair colourant composition.

17. A method as claimed in any one of the preceding claims wherein the composition is a hair conditioning composition comprising a hair conditioning agent.

18. A method as claimed in any one of Claims 1 to 17 wherein the composition is a shampoo composition further comprising a surfactant.

19. A method of producing a hair colouring composition as described in anyone of Claims 1 to 18 which comprises dispersing the clay in an aqueous liquid to form a dispersion and then bringing the agent into contact with the dispersion.

20. Method as claimed in Claim 19 wherein the clay is hydrated before the agent is added.

21. Use of a clay to reduce the amount that an agent capable of imparting a colour to hair colours skin relative to the amount that the agent colours hair, wherein the clay has a net charge at its surface and the agent has a net charge which is opposite from that of the clay.

22. Use as claimed in Claim 21, wherein the clay is an anionic clay and the agent is a cationic dye molecule.

23. Use as claimed in Claim 21, wherein the clay is a cationic clay and the agent is an anionic dye molecule.

## Patentansprüche

1. Haarfärbeverfahren, umfassend den Schritt der Auftragung auf das Haar einer Zusammensetzung zum Ausspülen, umfassend:
(i) einen Ton mit einer positiven oder negativen Nettoladung an seiner Oberfläche;
(ii) ein Mittel, das dem Haar Farbe verleihen kann; und
(iii) einen Duftstoff und/oder ein oberflächenaktives Mittel,
wobei das Mittel eine Nettoladung aufweist, die der Ladung an der Oberfläche des Tons entgegengesetzt ist.

2. Verfahren nach Anspruch 1, wobei der Ton eine Schichtstruktur ausweist.

3. Verfahren nach Anspruch 1 oder Anspruch 2, worin der Ton in Form einer Dispersion oder Suspension aus Tonteilchen vorliegt.

4. Verfahren nach einem der vorhergehenden Ansprüche, wobei zumindest so viel Ton enthalten ist, daß die Ladung an dem Mittel ausgeglichen wird.

5. Verfahren nach einem der vorhergehenden Ansprüche, worin das Gewichtsverhältnis von Ton zu Mittel im Bereich von 1 : 10 bis 1 : 0,5 liegt.

6. Verfahren nach Anspruch 5, worin das Gewichtsverhältnis von Ton zu Mittel im Bereich von 1 : 5 bis 1 : 0,7 liegt.

7. Verfahren nach einem der vorhergehenden Ansprüche, worin das Mittel ein molekulares Kation oder ein molekulares Anion ist.

8. Verfahren nach einem der vorhergehenden Ansprüche, worin der Ton ein anionischer Ton ist und das Mittel ein kationischer Farbstoff ist.

9. Verfahren nach Anspruch 8, worin der Ton aus der Klasse der Smectit-Tone ist.

10. Verfahren nach Anspruch 9, worin der Ton ein natürlicher, synthetischer oder chemisch modifizierter Ton, ausgewählt aus der Gruppe, bestehend aus Hektorit, Montmorillonit, Nontronit, Saponit, Beidelit, Sauconit und Gemischen davon, ist.

11. Verfahren nach Anspruch 10, worin der Ton synthetischer Hektorit ist.

12. Verfahren nach einem der Ansprüche 8 bis 11, worin das Mittel das Kation eines basischen Farbstoffes ist.

13. Verfahren einem der Ansprüche 1 bis 7, worin der Ton ein kationischer Ton ist und das Mittel ein anionischer Farbstoff ist.

14. Verfahren nach Anspruch 13, worin der Ton ein natürlicher oder synthetischer Schichtdoppelhydroxid-Ton ist.

15. Verfahren nach Anspruch 13 oder Anspruch 14, worin das Mittel das Anion eines sauren Farbstoffes ist.

16. Verfahren nach einem der vorhergehenden Ansprüche, worin die Zusammensetzung eine Haarfärbezusammensetzung ist.

17. Verfahren nach einem der vorhergehenden Ansprüche, worin die Zusammensetzung eine Haarpflegezusammensetzung ist, die ein Haarpflegemittel umfaßt.

18. Verfahren nach einem der Ansprüche 1 bis 17, worin die Zusammensetzung eine Shampoozusammensetzung ist, die ferner ein oberflächenaktives Mittel umfaßt.

19. Verfahren zur Herstellung einer Haarfärbezusammensetzung nach einem der Ansprüche 1 bis 18, das die Dispergierung des Tons in einer wässerigen Flüssigkeit zur Bildung einer Dispersion und das anschließende Inkontaktbringen des Mittels mit der Dispersion umfaßt.

20. Verfahren nach Anspruch 19, worin der Ton vor der Zugabe des Mittels hydratisiert wird.

21. Verwendung eines Tons zur Verringerung des Ausmaßes, zu dem ein Mittel, das dem Haar Farbe verleihen kann, die Haut verfärbt, bezogen auf das Ausmaß, zu dem das Mittel das Haar färbt, worin der Ton eine Nettoladung an seiner Oberfläche aufweist und das Mittel eine Nettoladung aufweist, die der des Tons entgegengesetzt ist.

22. Verwendung nach Anspruch 21, worin der Ton ein anionischer Ton ist und das Mittel ein kationisches Farbstoffmolekül ist.

23. Verwendung nach Anspruch 21, worin der Ton ein kationischer Ton ist und das Mittel ein anionisches Farbstoffmolekül ist.

## Revendications

1. Procédé de coloration capillaire comprenant l'étape d'application sur les cheveux d'une composition à rincer comprenant:
(i) une argile ayant une charge positive ou négative nette sur sa surface;
(ii) un agent capable de conférer une couleur aux cheveux ; et
(iii) un parfum et/ou un tensioactif,
dans lequel l'agent a une charge nette qui et opposée à la charge sur la surface de l'argile.

2. Procédé selon la revendication 1, dans lequel l'argile a une structure lamellaire.

3. Procédé selon la revendication 1 ou la revendication 2, dans lequel l'argile est présente sous la forme d'une dispersion ou suspension de particules d'argile.

4. Procédé selon l'une quelconque des revendications précédentes, qui contient au moins suffisamment d'argile pour équilibrer la charge sur l'agent.

5. Procédé selon l'une quelconque des revendications précédentes, dans lequel le rapport en poids de l'argile à l'agent est situé dans la plage allant de 1/10 à 1/0,5.

6. Procédé selon la revendication 5, dans lequel le rapport en poids de l'argile à l'agent est situé dans la plage allant de 1/5 à 1/0,7.

7. Procédé selon l'une quelconque des revendications précédentes, dans lequel l'agent est un cation moléculaire ou un anion moléculaire.

8. Procédé selon l'une quelconque des revendications précédentes, dans lequel l'argile est une argile anionique et l'agent est un colorant cationique.

9. Procédé selon la revendication 8, dans lequel l'argile appartient à la classe des argiles smectites.

10. Procédé selon la revendication 9, dans lequel l'argile est une argile naturelle, synthétique, ou chimiquement modifiée, du type choisi dans le groupe constitué par l'hectorite, la montmorillonite, la nontronite, la saponite, la beidélite, la sauconite et leurs mélanges.

11. Procédé selon la revendication 10, dans lequel l'argile est une hectorite synthétique.

12. Procédé selon l'une quelconque des revendications 8 à 11, dans lequel l'agent est le cation d'un colorant basique.

13. Procédé selon l'une quelconque des revendications 1 à 7, dans lequel l'argile est une argile cationique et l'agent est un colorant anionique.

14. Procédé selon la revendication 13, dans lequel l'argile est une argile de type hydroxyde double lamellaire naturelle ou synthétique.

15. Procédé selon la revendication 13 ou la revendication 14, dans lequel l'agent est l'anion d'un colorant acide.

16. Procédé selon l'une quelconque des revendications précédentes, dans lequel la composition est une composition de coloration capillaire.

17. Procédé selon l'une quelconque des revendications précédentes, dans lequel la composition est une composition de conditionnement des cheveux comprenant un agent de conditionnement des cheveux.

18. Procédé selon l'une quelconque des revendications 1 à 17, dans lequel la composition est une composition de shampooing comprenant en outre un tensioactif.

19. Procédé pour produire une composition de coloration capillaire telle que décrite dans l'une quelconque des revendications 1 à 18, qui comprend le fait de disperser l'argile dans un liquide aqueux pour former une dispersion et ensuite de mettre l'agent en contact avec la dispersion.

20. Procédé selon la revendication 19, dans lequel l'argile est hydratée avant que l'agent soit ajouté.

21. Utilisation d'une argile pour réduire la quantité en laquelle un agent capable de conférer une couleur aux cheveux colore la peau par rapport à la quantité en laquelle l'agent colore les cheveux, dans laquelle l'argile a une charge nette sur sa surface et l'agent a une charge nette qui est opposée à celle de l'argile.

22. Utilisation selon la revendication 21, dans laquelle l'argile est une argile anionique et l'agent est une molécule de colorant cationique.

23. Utilisation selon la revendication 21, dans laquelle l'argile est une argile cationique et l'agent est une molécule de colorant anionique.
